(19) **Europäisches Patentamt / European Patent Office / Office européen des brevets**

(11) **EP 4 301 343 B1**

(12) **EUROPEAN PATENT SPECIFICATION**

(45) Date of publication and mention
of the grant of the patent:
**19.11.2025 Bulletin 2025/47**

(21) Application number: **22711808.0**

(22) Date of filing: **04.03.2022**

(51) International Patent Classification (IPC):
**A61K 9/127** (2025.01)     **A61K 9/06** (2006.01)
**A61K 47/36** (2006.01)     **A61K 39/395** (2006.01)
**A61P 27/02** (2006.01)     **A61P 35/00** (2006.01)

(52) Cooperative Patent Classification (CPC):
**A61K 9/0048; A61K 9/0019; A61K 9/06;
A61K 9/127; A61K 31/573; A61K 39/3955;
A61K 47/36; A61P 1/00; A61P 27/02; A61P 35/00;
C07K 16/22;** A61K 2039/54

(86) International application number:
**PCT/US2022/018885**

(87) International publication number:
**WO 2022/187607 (09.09.2022 Gazette 2022/36)**

(54) **PHARMACEUTICAL COMPOSITIONS WITH REDUCED SIDE EFFECT AND METHODS OF USING THE SAME**

PHARMAZEUTISCHE ZUSAMMENSETZUNGEN MIT REDUZIERTEM NEBENEFFEKT UND VERFAHREN ZUR VERWENDUNG DAVON

COMPOSITIONS PHARMACEUTIQUES À EFFET SECONDAIRE RÉDUIT ET LEURS PROCÉDÉS D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.03.2021 US 202163157113 P**

(43) Date of publication of application:
**10.01.2024 Bulletin 2024/02**

(73) Proprietors:
• **TLC Biopharmaceuticals, Inc.**
  **South San Francisco CA 94080 (US)**
• **Taiwan Liposome Company, Ltd.**
  **Taipei City 11503 (TW)**

(72) Inventors:
• **SHIH, Sheue-Fang**
  **Taipei City, 11503 (TW)**
• **CHIU, Hsin-Yi**
  **Taipei City, 11503 (TW)**

(74) Representative: **Regimbeau**
  **20, rue de Chazelles**
  **75847 Paris Cedex 17 (FR)**

(56) References cited:
WO-A1-2017/075055     US-A1- 2014 105 960
US-A1- 2019 151 236     US-A1- 2021 059 980

**Description**

**BACKGROUND**

*Technical Field*

The present disclosure relates to a drug delivery system for delivery of an active agent to an eye. The present disclosure relates to pharmaceutical compositions having a reduced ocular side effect profile, and their uses in the ophthalmic field.

*Description of Related Art*

Conventional drug therapy for eye diseases is commonly achieved by the administration of solutions or suspensions. The limitations of these dosage forms include rapid clearance and undesired local toxicity.

Lipid-containing particles as drug delivery vehicles modify the pharmacokinetic profile of the drug, where liposomes usually enable sustained drug release, prolonged drug retention, and high administered doses with less frequent drug administration. Studies have demonstrated the enhanced drug retention and prolonged efficacy of the liposomal compositions in the eye (U.S. Patent No. 4,804,539, U.S. Patent No. 8,956,600, and M. Abrishami et al., Retina. 2009 May;29(5):699-703).

However, after administration, particularly via intravitreal injection, of a composition containing large unilamellar vesicles with high encapsulation efficiency to the vitreous humor, patients receiving said composition were reported to have blurred vision, which is associated with the vitreous clouding due to the lipid nanoparticles. It was found that the vision of an eye receiving an intravitreal injection of the indicated liposome-encapsulated ganciclovir declined initially owing to vitreous clouding, and the intravitreally injected liposome-encapsulated ganciclovir appeared as a whitish suspension located in the inferior part of the vitreous (S K Akula et al., Br J Ophthalmol. 1994 Sep; 78(9): 677-680). Further hydrogel compositions comprising liposomes are disclosed in US 2019/151236 A1 and US 2014/105960 A1.

Therefore, there remains an unmet need for a pharmaceutical composition for use in delivery of an active agent to an eye, which can achieve a prolonged therapeutic effect as well as reduced side effects, such as vitreous haze. The present disclosure addresses this need and other needs.

**SUMMARY**

The present disclosure provides a pharmaceutical composition for use in intravitreal administration, which comprises: a lipid mixture comprising one or more lipids (e.g., phospholipids); an effective amount of active agent or a pharmaceutically acceptable salt thereof; and a biocompatible hydrogel comprising hyaluronic acid, wherein the biocompatible hydrogel is present in the pharmaceutical composition at a concentration ranging from 0.01% (w/v) to 0.75% (w/v);
wherein the active agent is an anti-angiogenesis agent or an anti-inflammatory agent, and wherein the pharmaceutical composition has a viscosity of 15 to 220 mPa·S at 20°C. An ocular side effect caused by the delivery of the active agent in the pharmaceutical composition of the invention is reduced, compared to the ocular side effect of the same pharmaceutical composition in the absence of the biocompatible hydrogel (e.g., a reference pharmaceutical composition).

To improve the treatment of ophthalmic diseases and to obtain a desired administrability as well as a desired drug release profile, disclosed is a pharmaceutical composition for use in the delivery of an active agent to an eye. The pharmaceutical composition may include a mixture of a liposomal active agent and a biocompatible hydrogel. Thus, an enhanced treatment of ophthalmic diseases with desired pharmacokinetics as well as reduced side effect in the eye is achieved.

An exemplary biocompatible hydrogel according to the present disclosure comprises at least one polysaccharide, for example an unbranched mucopolysaccharide, including but not limited to, an anionic, non-sulfated glycosaminoglycan, such as hyaluronic acid (HA). Other polysaccharides which may be used include alginate, agarose, chitosan, collagen, chondroitin sulfate, gelatin, sodium cellulose sulfate, or mixtures thereof. The biocompatible hydrogel according to the present invention is present in a concentration ranging from 0.01% (w/v) to 0.75% (w/v) of the pharmaceutical composition. In some embodiments, the amount of the one or more lipids (e.g., phospholipids) according to the present disclosure ranges from about 2 $\mu$mol to about 200 $\mu$mol per mL of the pharmaceutical composition. The pharmaceutical composition according to the present disclosure has a suitable viscosity, in which the lipid nanoparticles would not disperse quickly in the vitreous humor and thus cause less vitreous clouding or haze initially after administration of the pharmaceutical composition. The present pharmaceutical composition also retains the administrability, e.g., injectability. In addition, the provided pharmaceutical composition also exhibits a reduced turbidity even after the lipid nanoparticles have dispersed completely, which efficiently ameliorates the vitreous haze caused by the administration of liposomes alone.

Meanwhile, the pharmaceutical composition according to the present disclosure offers sustained release of the active agent comparable to the same pharmaceutical composition in the absence of the biocompatible hydrogel. It is known that liposomes may modify the pharmacokinetics of the active agent which is a major concern of safety when developing a liposomal drug. The provided pharmaceutical composition shows a sustained release profile similar to that of the same pharmaceutical composition without the biocompatible hydrogel, and thus also maintains desired and prolonged efficacy without undermining safety.

Given the advantages described above, the provided pharmaceutical composition can improve patient outcomes and compliance.

In certain embodiments, the pharmaceutical composition according to the present disclosure is prepared by a process comprising: providing the lipid mixture and the active agent in a suspension; and mixing the suspension with a solution containing the biocompatible hydrogel, whereby a mixture of multilamellar vesicles and the biocompatible hydrogel forms.

The present disclosure also describes a method of delivering an active agent to an eye of a subject in need thereof, comprising administering a pharmaceutical composition in accordance with the present disclosure to the eye of the subject. In a particular embodiment, the step of administering a pharmaceutical composition in accordance with the present disclosure to the eye of the subject in need thereof comprises intravitreally administering the pharmaceutical composition to the vitreous humor of the eye of the subject. The method can achieve both a prolonged efficacy and a reduced turbidity in the vitreous humor after administration of the pharmaceutical composition according to the present disclosure, compared to that of a conventional composition with a prolonged releasing profile of the active agent.

The present disclosure also describes a pharmaceutical composition for use in the treatment of an eye disease. In particular, the present disclosure describes the use of the pharmaceutical composition in the manufacture of a medicament for the treatment of the eye disease where reduction of an ocular side effect is further improved by delivery of the active agent by the pharmaceutical composition according to the present disclosure.

Other objectives, advantages, and novel features of the disclosure will become apparent from the following detailed description when taken in conjunction with the accompanying drawings.

## BRIEF DESCRIPTION OF THE DRAWINGS

Figs. 1A to 1E are photographs showing the dispersion behaviors of the liposome-DSP (dexamethasone sodium phosphate) composition and HA/liposome-DSP compositions in saline at 37 °C.

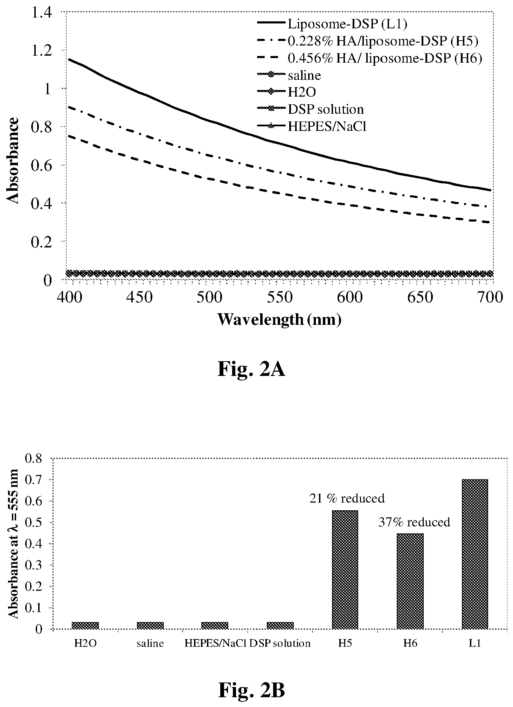

Figs. 2A and 2B are graphs depicting the turbidity measurements of the dispersed liposome-DSP composition and HA/liposome-DSP compositions in saline. Fig. 2A shows that the liposome-DSP and HA/liposome-DSP compositions were both well-dispersed in saline. The homogeneous solutions were added to a 96-well plate and the absorbance measured at $\lambda = 400$ to 700 nm. The turbidity of the samples was then calculated from the absorbance (the higher the absorbance of light, the higher the turbidity). The blank solutions (saline, $H_2O$, DSP solution and HEPES/NaCl) were also measured as references. Fig. 2B demonstrates absorbance of the samples at $\lambda = 555$ nm.

Fig. 3 is a line graph showing *in vitro* release of DSP from the liposome-DSP composition (sample L1) and the HA/liposome-DSP composition (sample H3) in saline. The total amounts of the phospholipids and DSP in the samples of the two groups were the same.

## DETAILED DESCRIPTION OF THE EMBODIMENTS

*Definition*

As employed above and throughout the disclosure, the following terms, unless otherwise indicated, shall be understood to have the following meanings.

As used herein, the singular forms "a", "an" and "the" include the plural reference unless the context clearly indicates otherwise.

All numbers herein may be understood as modified by "about," which, when referring to a measurable value such as an amount, a temporal duration, and the like, is meant to encompass variations of $\pm 10\%$, preferably $\pm 5\%$, more preferably $\pm 1\%$, and even more preferably $\pm 0.1\%$ from the specified value, as such variations are appropriate to obtain a desired amount of liposomal composition, unless otherwise specified.

The term "treating," "treated," or "treatment" as used herein includes, but not limited to preventive (e.g., prophylactic), palliative, and/or curative uses or results.

The term "effective amount" as used herein denotes a dose of the pharmaceutical composition that is sufficient to reduce, prevent or eliminate the symptoms or signs of a disease, syndrome, disorder or condition.

The term "pharmaceutically acceptable salts" of the active agent of the present disclosure are salts of an acidic active agent formed with bases, namely base addition salts such as alkali and alkaline earth metal salts, e.g., sodium, lithium, potassium, calcium, magnesium, as well as ammonium salts, e.g., ammonium, trimethyl-ammonium, diethyl-lammonium, and tris-(hydroxymethyl)-methyl-ammonium salts. Similarly acid addition salts, such as of mineral acids, organic carboxylic and organic sulfonic acids, e.g., hydrochloric acid, methanesulfonic acid, maleic acid, are also possible provided to a basic active agent.

The term "subject" includes a vertebrate having ophthalmic diseases affecting ocular function. In some embodiments, the subject is a warm-blooded animal, such as mammals, including humans.

In certain embodiments, the phospholipid amount or concentration of liposome and liposomal composition could be determined by assaying the phosphorus content of liposome and liposomal drug samples using a phosphorus assay (adapted from G. Rouser et al., Lipids 1970, 5, 494-496).

As used herein, the term "% (w/v)" refers to percent weight/volume, which is defined as mass of a solute in grams present in 100 ml of solution.

As used herein, the term "ocular side effect" refers to active agent-induced side effects including, but not limited to, elevated ocular pressure, glaucoma, cataract formation, delayed wound healing, increased susceptibility to infection, vitreous haze and vision problems. As used herein, the term "vitreous haze" refers to the appearance of diffused turbidity or milkiness in the vitreous humor which may cause vision problems. Eye examination for vitreous humor can be done using a direct or indirect ophthalmoscope for speculating opacification via arbitrary index. As much of the fundus as is visible, including the retina, macula, fovea, vessels, and optic disk and its margins, is examined to see the entire fundus (i.e., to see a peripheral retinal detachment).

As used herein, the term "turbidity" means cloudiness or haziness of a fluid caused by suspended particles. The turbidity could be, but not limited to be measured using a spectrophotometer.

### Lipid Mixture

The lipid mixture of the pharmaceutical composition provided herein comprises at least one lipids, for example, but not limited to: a phospholipid or a mixture of phospholipids. The lipid mixture includes, but not limited to, film, cake, granules, powders or solution before being added to the pharmaceutical composition.

In one embodiment, the phospholipid or the mixture of phospholipids, with or without cholesterol, are pre-formed into liposomes before further processing into a lipid mixture.

In another embodiment, the phospholipid or mixture of phospholipids, with or without cholesterol, are not pre-formed into liposomes before further processing into a lipid mixture.

The lipid mixture in accordance with the present disclosure can be prepared from a variety of lipids capable of either forming or being incorporated into a unilayer or bilayer structure. The lipids used in the present disclosure include one or more phospholipids, including but are not limited to, phosphatidylcholine (PC), phosphatidylglycerol (PG), phosphatidylethanolamine (PE), phosphatidylserine (PS), phosphatidic acid (PA), phosphatidylinositol (PI) or combinations thereof.

In some embodiments, the lipid mixture comprises egg phosphatidylcholine (EPC), egg phosphatidylglycerol (EPG), egg phosphatidylethanolamine (EPE), egg phosphatidylserine (EPS), egg phosphatidic acid (EPA), egg phosphatidylinositol (EPI), soy phosphatidylcholine (SPC), soy phosphatidylglycerol (SPG), soy phosphatidylethanolamine (SPE), soy phosphatidylserine (SPS), soy phosphatidic acid (SPA), soy phosphatidylinositol (SPI) or combinations thereof. In another embodiments, the lipid mixture comprises dipalmitoylphosphatidylcholine (DPPC), 1,2-dioleoyl-sn-glycero-3- phosphatidylcholine (DOPC), dimyristoylphosphatidylcholine (DMPC), dipalmitoylphosphatidylglycerol (DPPG), dioleoylphosphatidylglycerol (DOPG), dimyristoylphosphatidylglycerol (DMPG), hexadecylphosphocholine (HEPC), hydrogenated soy phosphatidylcholine (HSPC), distearoylphosphatidylcholine (DSPC), distearoylphosphatidylglycerol (DSPG), dioleoylphosphatidylethanolamine (DOPE), palmitoylstearoylphosphatidylcholine (PSPC), palmitoylstearoylphosphatidylglycerol (PSPG), monooleoylphosphatidylethanolamine (MOPE), 1-palmitoyl-2-oleoyl-sn-glycero-3-phosphatidylcholine (POPC), polyethyleneglycol distearoylphosphatidylethanolamine (PEG-DSPE), dipalmitoylphosphatidylserine (DPPS), 1,2-dioleoyl-sn-glycero-3-phosphatidylserine (DOPS), dimyristoylphosphatidylserine (DMPS), distearoylphosphatidylserine (DSPS), dipalmitoylphosphatidic acid (DPPA), 1,2-dioleoyl-sn-glycero-3-phosphatidic acid (DOPA), dimyristoylphosphatidic acid (DMPA), distearoylphosphatidic acid (DSPA), dipalmitoylphosphatidylinositol (DPPI), 1,2-dioleoyl-sn-glycero-3- phosphatidylinositol (DOPI), dimyristoylphosphatidylinositol (DMPI), distearoylphosphatidylinositol (DSPI), or combinations thereof.

In some embodiments, the lipid mixture comprises a first phospholipid and a second phospholipid. In some embodiments, the first phospholipid is selected from the group consisting of EPC, EPE, SPC, SPE, DPPC, DOPC, DMPC, HEPC, HSPC, DSPC, DOPE, PSPC, MOPE, POPC; and the second phospholipid is selected from the group consisting of PG, PS, PA, PI, EPG, EPS, EPA, EPI, SPG, SPE, SPS, SPA, SPI, DPPG, DOPG, DMPG, DSPG, PSPG, DPPS, DOPS, DMPS, DSPS, DPPA, DOPA, DMPA, DSPA, DPPI, DOPI, DMPI, DSPI, and a hydrophilic polymer with a long chain of highly hydrated flexible neutral polymer attached to a phospholipid molecule. Examples of the hydrophilic polymer include, but are not limited to, polyethylene glycol (PEG) with a molecular weight about 2,000 to about 5,000 daltons, methoxy PEG (mPEG), ganglioside $GM_1$, polysialic acid, polylactic acid (also termed polylactide), polyglycolic acid (also termed polyglycolide), polylacticpolyglycolic acid, polyvinyl alcohol, polyvinylpyrrolidone, polymethoxazoline, polyethyloxazoline, polyhydroxyethyloxazoline, polyhydroxypropyloxazoline, polyaspartamide, polyhydroxypropyl methacrylamide, polymethacrylamide, polydimethylacrylamide, polyvinylmethylether, polyhydroxyethyl acrylate, derivatized celluloses such as hydroxymethylcellulose or hydroxyethylcellulose and synthetic polymers.

In a particular embodiment, the lipid mixture further comprises a sterol. Sterol used in the present disclosure is not particularly limited, with examples thereof including cholesterol, phytosterol (sitosterol, stigmasterol, fucosterol, spinasterol, brassicasterol, and the like), ergosterol, cholestanone, cholestenone, coprostenol, cholesteryl-2'-hydroxyethyl ether, and cholesteryl-4'-hydroxybutyl ether. The sterol component of the lipid mixture, when present, can be any of those sterols conventionally used in the field of lipid particle preparation.

In a more particular embodiment, the lipid mixture further comprises a cholesterol. In some embodiments, the lipid mixture comprises about 10 mole % to about 33 mole % of cholesterol, about 15 mole % to about 30 mole % of cholesterol, about 18 mole % to about 28 mole % of cholesterol, or about 20 mole % to about 25 mole % of cholesterol.

In a particular embodiment, the lipid mixture comprises the first phospholipid, the second phospholipid and the sterol at a mole percent ratio of 29.5% to 90%:3% to 37.5%: 10 % to 33%.

In a particular embodiment, the first phospholipid is DOPC, POPC, SPC, or EPC and the second phospholipid is PEG-DSPE, or DOPG.

In a more particular embodiment, the lipid mixture comprises DOPC and DOPG.

In one embodiment, the lipid mixture is substantially free of fatty acid or cationic lipid (i.e., a lipid carrying a net positive charge a physiological pH).

In one embodiment, the lipid mixture optionally comprises a lipid-conjugate of an antibody or a peptide that acts as a targeting moiety to enable liposomes thereof to specifically bind to a target cell bearing a target molecule. Examples of the target molecules include, but are not limited to, TNF-$\alpha$ and B cell surface antigen, such as CD20. Other antigens, such as CD19, HER-3, GD2, Gp75, CS1 protein, mesothelin, c-Myc, CD22, CD4, CD44, CD45, CD28, CD3, CD123, CD138, CD52, CD56, CD74, CD30, Gp75, CD38, CD33, GD2, vascular endothelial growth factor (VEGF), or transforming growth factor (TGF) may also be used.

## Lipid-containing particles

As used herein, "lipid-containing particle" or "lipid nanoparticle (LNP)" in accordance with the present disclosure includes, but is not limited to, a population of particles comprising a lipid and additionally other biocompatible excipients for forming a vehicle for delivery of an active agent. A variety of lipid-containing nanoparticles have been demonstrated, such as liposomes, micelles, or other lipid-containing nanoparticles. Method of producing the lipid nanoparticle formulation can influence and/or dictate distribution of certain components within the lipid nanoparticles, and that this distribution can influence and/or dictate physical (e.g., stability) and/or biological (e.g. efficacy, intracellular delivery, immunogenicity) properties of the lipid nanoparticles as desired.

As used herein, the term "lipid nanoparticle" includes, but is not limited to, a delivery vehicle comprising one or more lipids and formulated into a form of particles having an average size or diameter of nanometer to micrometer to deliver one or more active agents to a subject in need thereof. Examples of suitable lipids include cationic lipids, non-cationic lipids, and PEG-modified lipids.

In a group of embodiments, the lipid nanoparticles in accordance with the present disclosure are liposomes. The liposome in accordance with the present disclosure includes, but is not limited to, a lipid bilayer surrounding an internal aqueous agent-carrying component. Non-limiting examples of liposomes include small unilamellar vesicles (SUV), large unilamellar vesicles (LUV), multivesicular liposome (MVL) and multi-lamellar vesicles (MLV).

The liposomes prepared in this disclosure can be generated by conventional techniques used to prepare vesicles. These techniques include the ether injection method (Deamer et al., Acad. Sci. (1978) 308: 250), the surfactant method (Brunner et al., Biochim. Biophys. Acta (1976) 455: 322), the freeze-thaw method (Pick et al., Arch. Biochim. Biophys. (1981) 212: 186), the reverse-phase evaporation method (Szoka et al., Biochim. Biophys. Acta. (1980) 601: 559 71), the ultrasonic treatment method (Huang et al., Biochemistry (1969) 8: 344), the ethanol injection method (Kremer et al., Biochemistry (1977) 16: 3932), the extrusion method (Hope et al., Biochim. Biophys. Acta (1985) 812:55 65), the French press method (Barenholz et al., FEBS Lett. (1979) 99: 210) and methods detailed in Szoka, F., Jr., et al., Ann. Rev. Biophys. Bioeng. 9:467 (1980). All of the above processes are basic technologies for the formation of vesicles. After sterilization, the pre-formed liposomes can be placed aseptically into a container and then lyophilized to form a powder or a cake. In the embodiment where the lipid mixture comprises pre-formed liposomes, said liposomes can be obtained by solvent injection method and followed by lyophilization to form the lipid mixture. The lipid mixture optionally comprises one or more bulking agent. In one embodiment, the lipid mixture further comprises one or more buffering agents.

The bulking agents include, but are not limited to, polyols or sugar alcohols such as mannitol, glycerol, sorbitol, dextrose, sucrose, and/or trehalose; amino acids such as histidine, and glycine. One preferred bulking agent is mannitol.

The buffering agents include, but are not limited to, sodium phosphate monobasic dihydrate and sodium phosphate dibasic anhydrous.

In the embodiment where the lipid mixture comprises lipids that are not pre-formed into liposomes, the lipid mixture can be prepared by dissolving in a suitable organic solvent, including, but not limited to, ethanol, methanol, t-butyl alcohol, ether and chloroform, and drying by heating, vacuum evaporation, nitrogen evaporation, lyophilization, or other conventional means of solvent removal.

## *Active agent*

The active agent of the preset disclosure can be mixed either in $ddH_2O$ or a suitable buffer as a solution containing the active agent for suspending the lipid mixture to obtain the pharmaceutical composition according to the present disclosure. In some embodiments, the active agent is not covalently bound to a lipid such as phospholipid or a fatty acid, such as palmitate.

In one aspect, the active agent refers to a substance that induces a desired pharmacological or physiological effect, which alleviates a symptom of or prevents development of a disease or disorder.

The active agent that can be used in the pharmaceutical composition disclosed herein comprises one or more of an anti-angiogenesis agent, an anti-inflammatory agent, an immunosuppressive agent, an antimicrobial agent, or an antiviral agent. According to the present invention, the active agent is one or more of an anti-angiogenesis agent or an anti-inflammatory agent.

Examples of the anti-angiogenesis agent include, but are not limited to, bevacizumab (Avastin®, Genentech), ranibizumab (Lucentis®, Genentech), aflibercept (Eylea®, Regeneron), brolucizumab (Beovu®, Novartis), and pegaptanib (Macugen®, Valeant).

In one embodiment, the anti-inflammatory agents include, but are not limited to, steroid, particularly ocular steroid, a derivative thereof, a pharmaceutically acceptable salt thereof, or a prodrug thereof. The steroid useful in the present disclosure includes any naturally occurring steroid hormones, synthetic steroids and their derivatives. In a particular embodiment, the anti-inflammatory agent is corticosteroid. Examples of the steroid include, but are not limited to, cortisone, hydrocortisone, hydrocortisone acetate, tixocortol pivalate, fluocinolone, prednisolone, methylprednisolone, prednisone, triamcinolone acetonide, triamcinolone, mometasone, amcinonide, budesonide, desonide, fluocinonide, fluocinolone acetonide, halcinonide, betamethasone, betamethasone sodium phosphate, dexamethasone, dexamethasone sodium phosphate (DSP), fluocortolone, hydrocortisone-17-butyrate, hydrocortisone-17-valerate, alclometasone dipropionate, betamethasone valerate, betamethasone dipropionate, prednicarbate, clobetasone-17-butyrate, clobetasol-17-propionate, fluocortolone caproate, fluocortolone pivalate, fluprednidene acetate, difluprednate, loteprednol, fluorometholone, medrysone rimexolone, beclomethasone, cloprednol, cortivazol, deoxycortone, difluorocortolone, fluclorolone, fludrocortisone, flumethasone, flunisolide, flurandrenolone, meprednisone, methylprednisolone and paramethasone. In certain embodiments, the ocular steroid is a water soluble steroid. In certain embodiments, the water soluble steroid is DSP.

Examples of the immunosuppressive agent include, but are not limited to, methotrexate, azathioprine, mycophenolate mofetil, cyclosporine, tacrolimus, voclosporin, cyclophosphamide, chlorambucil, etanercept, infliximab, adalimumab, rituximab, abatacept, anakinra, and daclizumab.

Examples of the antimicrobial agent include, but are not limited to, vancomycin, ceftazidime, amikacin, amphotericin, and voriconazole.

Examples of the antiviral agent include, but are not limited to, ganciclovir, foscarnet, cidofovir, and fomivirsen.

In some embodiments, the active agent is selected from the group consisting of bevacizumab, ranibizumab, aflibercept, brolucizumab, pegaptanib, cortisone, hydrocortisone, hydrocortisone acetate, tixocortol pivalate, fluocinolone, prednisolone, methylprednisolone, prednisone, triamcinolone acetonide, triamcinolone, mometasone, amcinonide, budesonide, desonide, fluocinonide, fluocinolone acetonide, halcinonide, betamethasone, betamethasone sodium phosphate, dexamethasone, dexamethasone sodium phosphate (DSP), fluocortolone, hydrocortisone-17-butyrate, hydrocortisone-17-valerate, alclometasone dipropionate, betamethasone valerate, betamethasone dipropionate, prednicarbate, clobetasone-17-butyrate, clobetasol-17-propionate, fluocortolone caproate, fluocortolone pivalate, fluprednidene acetate, difluprednate, loteprednol, fluorometholone, medrysone rimexolone, beclomethasone, cloprednol, cortivazol, deoxycortone, difluorocortolone, fluclorolone, fludrocortisone, flumethasone, flunisolide, flurandrenolone, meprednisone, methylprednisolone and paramethasone.

In some embodiments, the active agent is an antagonist specific to VEGF, which can be an antibody specific to VEGF, a VEGF receptor, a nucleic acid binding to VEGF, or a small molecule that interferes with the interaction between VEGF and its cognate receptor and blocks the VEGF signaling pathway. The term "antibody" used herein refers to a naturally-occurring immunoglobulin, a functional fragment thereof, or a genetically modified immunoglobulin, such as humanized antibody, chimeric antibody, or fully human antibody.

In some embodiments, the therapeutic agent is an antibody specific to VEGF. Such antibody specific to VEGF may be a complete antibody molecule having full length heavy and light chains or a functional fragment, such as Fab, Fab', $F(ab)_2$, $F(ab')_2$, scFv, di-scFv, scFv-Fc, single domain antibody, diabody, and triabody.

In one example, the imaging agent refers to a substance useful for imaging applications. Examples of the imaging agent include, but are not limited to, radioconjugate, fluorescent agents, gadolinium-DTPA (Gd-DTPA), iron oxide, manganese oxide, and quantum dots.

*Biocompatible hydrogel*

The term "biocompatible hydrogel" as used herein refers to a substance which is not toxic to living cells and in which polymers form a network structure (e.g., by chemical bonding) and a large amount of water is retained in the network.

In an example, the biocompatible hydrogel comprises a polymer selected from the group consisting of polysaccharide, polyphosphazene, poly(acrylic acids), poly(methacrylic acids), poly(alkylene oxides), poly(vinyl acetate), polyvinylpyrrolidone (PVP), and copolymers and blends thereof.

In one example, the biocompatible hydrogel comprises a polysaccharide selected from the group consisting of hyaluronic acid, alginate, agarose, chitosan, collagen, chondroitin sulfate, gelatin, sodium cellulose sulfate, and combinations thereof.

Exemplary biocompatible hydrogels according to the present disclosure include, but are not limited to, polysaccharides, particularly unbranched mucopolysaccharides. In certain embodiments, the biocompatible hydrogel according to the present disclosure is O-linked or N-linked glycosaminoglycan, particularly non-sulfated glycosaminoglycan. In certain embodiments, the biocompatible hydrogel according to the present disclosure includes, but is not limited to, an anionic, non-sulfated glycosaminoglycan.

In the present invention, the biocompatible hydrogel comprises hyaluronic acid.

In some embodiments, the biocompatible hydrogel has a molecular weight of about 4 kDa to about 8000 kDa, optionally about 4 kDa to about 7000 kDa, optionally about 50 kDa to about 8000 kDa, optionally about 50 kDa to about 7000 kDa, optionally about 50 kDa to about 6000 kDa, optionally about 50 kDa to about 5000 kDa, optionally about 50 kDa to about 4000 kDa, optionally about 50 kDa to about 3000 kDa, optionally about 50 kDa to about 2500 kDa, optionally about 50 kDa to about 2000 kDa, optionally about 100 kDa to about 2500 kDa, optionally about 100 kDa to about 2000 kDa, optionally about 1500 kDa to about 1800 kDa.

*The pharmaceutical composition*

The pharmaceutical composition of the present disclosure is suitable for delivery of an active agent to an eye and comprises a lipid mixture comprising one or more lipids (e.g., phospholipids); an effective amount of the active agent or a pharmaceutically acceptable salt thereof; and a biocompatible hydrogel comprising hyaluronic acid, wherein the biocompatible hydrogel is present in the pharmaceutical composition at a concentration ranging from 0.01% (w/v) to 0.75% (w/v), the active agent is an anti-angiogenesis agent or an anti-inflammatory agent, and the pharmaceutical composition has a viscosity of 15 to 220 mPa·S at 20°C ; wherein the ocular side effect of said pharmaceutical composition is reduced, compared to the ocular side effect of a reference pharmaceutical composition without the biocompatible hydrogel. The reference pharmaceutical composition includes, but is not limited to, the one or more lipids, and the active agent or a pharmaceutically acceptable salt thereof of the present disclosure but in the absence of the biocompatible hydrogel (where the reference pharmaceutical composition causes the ocular side effect). After intravitreal administration of the present pharmaceutical composition, the ocular side effect is reduced by about 10% to about 100%, about 10% to 40%, or about 20% to 40%, particularly by at least 10%, at least 15%, at least 20%, at least 25%, at least 30%, at least 35%, at least 40%, at least 45% and at least 50%, compared to that of a reference pharmaceutical composition in the absence of the biocompatible hydrogel.

In one embodiment, the amount of the one or more lipids (e.g., phospholipids) ranges from about 2 $\mu$mol to about 200 $\mu$mol per mL of the pharmaceutical composition, optionally from about 10 $\mu$mol to about 200 $\mu$mol per mL of the pharmaceutical composition, optionally from about 10 $\mu$mol to about 150 $\mu$mol per mL of the pharmaceutical composition, optionally from about 20 $\mu$mol to about 150 $\mu$mol per mL of the pharmaceutical composition, optionally from about 20 $\mu$mol to about 125 $\mu$mol per mL of the pharmaceutical composition, optionally from about 30 $\mu$mol to about 125 $\mu$mol per mL of the pharmaceutical composition, optionally from about 30 $\mu$mol to about 100 $\mu$mol per mL of the pharmaceutical composition, optionally from about 40 $\mu$mol to about 100 $\mu$mol per mL of the pharmaceutical composition, optionally from about 50 $\mu$mol to about 100 $\mu$mol per mL of the pharmaceutical composition, optionally from about 60 $\mu$mol to about 100 $\mu$mol per mL of the pharmaceutical composition.

In the present invention, the biocompatible hydrogel is present in a concentration ranging from 0.01% (w/v) to 0.75% (w/v), optionally about 0.05% (w/v) to about 0.75% (w/v), optionally about 0.01% (w/v) to about 0.6% (w/v), optionally about 0.05% (w/v) to about 0.6% (w/v), optionally about 0.01% (w/v) to about 0.5% (w/v), optionally about 0.05% (w/v) to about 0.5% (w/v), optionally about 0.06% (w/v) to about 0.5% (w/v), optionally about 0.07% (w/v) to about 0.5% (w/v), optionally about 0.08% (w/v) to about 0.5% (w/v), optionally about 0.09% (w/v) to about 0.5% (w/v), optionally about 0.0912% (w/v) to about 0.456% (w/v) of the pharmaceutical composition.

In one embodiment, the pharmaceutical composition is prepared by a process comprising: providing the lipid mixture and the active agent in a suspension; and mixing the suspension with a solution containing the biocompatible hydrogel, whereby a mixture of lipid-containing particles and the biocompatible hydrogel forms.

In a particular embodiment, said suspension is prepared by reconstituting a lyophilized lipid cake comprising the lipid mixture with an aqueous solution comprising the active agent. In another particular embodiment, said suspension is prepared by reconstituting a lyophilized combination of the lipid mixture and the active agent with an aqueous solution.

In an embodiment, the step of providing the lipid mixture and the active agent in a suspension comprises reconstituting the lipid mixture and the active agent in an aqueous solution to form lipid nanoparticles in the suspension. In the mixing step, a mixture of lipid nanoparticles and the biocompatible hydrogel forms in the pharmaceutical composition. After administration of the pharmaceutical composition to a subject in need thereof, the release profile of the active agent of the pharmaceutical composition is sustained in the vitreous humor of the subject without causing haze. In some embodiments, the lipid nanoparticles include multi-lamellar vesicles and a portion of the active agent is associated with the multi-lamellar vesicles.

In one example, the pharmaceutical composition is suitable for use in the delivery of an active agent to an eye via intravitreal injection, subconjunctival injection, subretinal injection, intracameral injection or topical administration. In a particular embodiment of the invention, the pharmaceutical composition is for use in intravitreal injection.

In one embodiment, the ocular side effect comprises vitreous haze. Vitreous haze may be observed after administration of a liposomal composition to the vitreous humor of an eye where the composition comprises a lipid mixture comprising one or more phospholipids; and an effective amount of the active agent or a pharmaceutically acceptable salt thereof.

The pharmaceutical composition of the present disclosure may have a greater viscosity than a reference pharmaceutical composition without the biocompatible hydrogel. The lipid nanoparticles together with the biocompatible hydrogel in the formed mixture of the present pharmaceutical composition are retained in the vitreous humor and lead to a reduction in vitreous haze initially after intravitreal injection of the pharmaceutical composition, compared to a reference ophthalmic pharmaceutical composition having the same components but without the biocompatible hydrogel. In particular, the reference ophthalmic pharmaceutical composition may be a suspension of lipid nanoparticles. In another aspect, after intravitreal administration, the present pharmaceutical composition is retained in the vitreous humor of a subject in need thereof for a retention time, which may be after the lipid nanoparticles, e.g. multi-laminar vesicles, are well-dispersed in the vitreous humor. After administration of the present pharmaceutical composition, the vitreous humor may show a lower turbidity relative to that of one administered with the reference ophthalmic pharmaceutical composition. In one embodiment, the pharmaceutical composition of the invention is for use in reducing vitreous haze caused by intravitreal injection of a liposomal composition into vitreous humor of an eye of a subject in need thereof, wherein the liposomal composition comprises the active agent without the biocompatible hydrogel.

### *Method of treating ophthalmic diseases or disorders*

The pharmaceutical composition according to the present disclosure is suitable for use in the treatment of ophthalmic diseases or disorders.

In one embodiment, the subject in need thereof according to the present disclosure is suffering from at least one ophthalmic disease or disorder. Examples of the ophthalmic disease or disorder include, but are not limited to, macular degeneration, cystoid macular edema (CME), diabetic maculopathy, proliferative diabetic retinopathy (PDR), diabetic macular edema, retinal vein occlusions (RVO), neovascular glaucoma (NVG), retinal vascular tumors, choroidal hemangiomas, choroidal melanoma, vasoproliferative ocular tumor, Iris melanoma, radiation retinopathy, central serous chorioretinopathy (CSR), retinopathy of prematurity (ROP), endophthalmitis, uveitis, and retinitis.

In one embodiment, administering the pharmaceutical composition comprises intravitreally administering the pharmaceutical composition to the vitreous humor.

One example of this disclosure is a method of delivering an active agent to the vitreous humor of a subject in need thereof and reducing the ocular side effect of a liposomal composition, comprising administering a pharmaceutical composition as described above.

In one embodiment, the ocular side effect comprises vitreous haze. An increase in vitreous turbidity is observed after administration of a liposomal composition without a biocompatible hydrogel to the vitreous humor. However, administering the present pharmaceutical composition to the vitreous humor reduces the vitreous turbidity, compared to administering the same pharmaceutical composition in the absence of the biocompatible hydrogel.

In certain embodiments, the pharmaceutical composition in accordance with the present disclosure has a phospholipid concentration ranging from about 50 mM to about 150 mM and the biocompatible hydrogel at an amount of about 0.05% to 1% (w/v), about 0.1% to 0.9% (w/v) or about 0.2% to 0.5% (w/v). After intravitreal administration of the pharmaceutical composition, the vitreous turbidity is reduced by about 10% to about 100%, about 10% to 40%, or about 20% to 40%, particularly by at least 10%, at least 15%, at least 20%, at least 25%, at least 30%, at least 35%, at least 40%, at least 45% and at least 50%, compared to that of a reference pharmaceutical composition in the absence of the biocompatible hydrogel. The viscosity of the pharmaceutical composition in accordance with the present disclosure is 15 to 220 mPa·S at 20°C.

The following examples further illustrate the present disclosure. These examples are intended merely to be illustrative of the present disclosure and are not to be construed as being limiting.

## EXAMPLES

The following examples illustrate the preparation and properties of certain embodiments of the present disclosure.

### Example 1 Preparation of a liposome composition for drug delivery

### A. Preparation of a lipid cake

A lipid mixture of one or more lipids, including DOPC, DOPG and cholesterol, as formed at a molar ratio of 67.5 : 7.5 : 25 (DOPC : DOPG : cholesterol) and dissolved in absolute ethanol at about 40 °C. A tabletop ultrasonic bath was used for lipid dissolution. The dissolved lipid solution was then added to a 1 mM of sodium phosphate solution to form a pro-liposome suspension, followed by extrusion of the pro-liposome suspension through a polycarbonate membrane with a pore size of 0.2 $\mu$m with 6 to 10 extrusion passes to form a liposome suspension. The liposome suspension was dialyzed and concentrated by a tangential flow filtration system (Spectrum® Hollow Fiber Filter Modules; MIDIKROS membrane: mPES, 100 kDa, 115 cm$^2$) against 1 mM of sodium phosphate solution and then sterilized using a 0.2 $\mu$m sterile filter to obtain a sterilized liposome suspension. The phospholipid concentration of the sterilized liposome suspension was quantified by a phosphorus assay. The sterilized liposome suspension was subsequently formulated with a cryoprotectant, 2 w/w% mannitol (aseptic), and filled aseptically into vials for lyophilization to form a lipid cake.

### B. Reconstitution of the lipid cake to form a liposome-drug composition

The liposomal active agent composition (liposome-drug composition) comprising the lipid mixture can be prepared by reconstitution of the lipid cake (prepared in Example 1A) with an active agent, such as a therapeutic agent or an imaging agent. In the present example, exemplary active agents are dexamethasone sodium phosphate (DSP) and bevacizumab.

I. Liposome-DSP composition

The liposomal DSP composition (also denoted as liposome-DSP composition) was prepared by reconstitution of the lipid cake (prepared in Example 1A) with a DSP solution, which comprises DSP (13.2 mg/mL) and sodium citrate (4.6 mg/mL) in an aqueous phase. The phospholipid concentration of the reconstituted liposome-DSP composition is 100 mM or 167 mM depending on the added volume of DSP solution.

II. Liposome-bevacizumab composition

The liposomal bevacizumab (an antibody specific to VEGF) composition (liposome-bevacizumab composition) was prepared by reconstitution of the lipid cake (prepared in Example 1A) with a bevacizumab solution comprising a suitable amount of bevacizumab in an aqueous phase. The bevacizumab concentration of the reconstituted liposome-bevacizumab composition can be, for example, 25 mg/mL.

### Example 2 Preparation of hydrogel solutions

The hydrogel solution in the present example was an aqueous solution comprising a biocompatible hydrogel which was prepared as described below, where the biocompatible hydrogel comprised hyaluronic acid. Hyaluronic acid powder (Hyaluronic acid sodium salt from *Streptococcus equi,* Sigma) was dissolved in ddH$_2$O (Milli-Q purification system, Millipore) or buffers (10 mM HEPES/145 mM NaCl, 10 mM L-histidine/145 mM NaCl or PBS (Gibco®/Thermo Fisher Scientific), etc.) at an indicated concentration (see Table 1), and stirred at 4 °C overnight for a comprehensive dissolution and cross-linking of HA.

The buffers for HA dissolution were prepared as follows:

(1) <u>10 mM HEPES/145 mM</u> NaCl: dissolving 119.15 mg of HEPES and 423.69 mg of NaCl in 50 mL of ddH$_2$O and adjusting the pH value to 7.4 using NaOH and/or HCl.

(2) <u>10 mM L-histidine/145 mM</u> NaCl: dissolving 77.75 mg of L-histidine and 423.69 mg of NaCl to 50 mL of ddH$_2$O and the pH to about 7.56.

**Table 1 HA solutions**

| HA concentration (w/v %) | Buffer for HA dissolution |
|---|---|
| 0.228 | ddH$_2$O |
| 0.57 | |
| 0.228 | HEPES/NaCl |
| 0.57 | |
| 1.14 | |
| 1.14 | L-histidine/NaCl |
| 2.28 | |
| 5.7 | |
| 11.4 | |
| 0.57 | PBS |
| 1.14 | |

**Example 3 Preparation of hydrogel/liposome-drug compositions**

The hydrogel solution and reconstituted liposome-drug composition were mixed to obtain a pharmaceutical composition comprising hydrogel and the liposomal active agent (also denoted as hydrogel/liposome-drug composition).

I. Hydrogel/liposome-DSP composition (HA/liposome-DSP composition)

The HA solution and reconstituted liposome-DSP composition as prepared by the method according to the previous examples were mixed in the volume ratio of 2 : 3 to form the HA/liposome-DSP composition. The final contents of HA and phospholipid in the HA/liposome-DSP compositions are listed in Table 4.

II. Hydrogel/liposome-bevacizumab composition (HA/liposome-bevacizumab composition)

The HA solution and reconstituted liposome-bevacizumab composition were mixed to form the HA/liposome-bevacizumab composition.

**Example 4 Viscosity determination and injectability assessment of the hydrogel/liposome-drug composition**

The viscosities of the samples were determined by a viscometer equipped with a HVROC-T/Temperature controller (μVISC™/HVROC-S, RheoSense). Before measurement, the temperature of the viscometer was set to 20 °C or 37 °C and balanced until steady state. 400 μL of the sample was loaded into a disposable viscometer pipette and mounted to the viscometer for measurement. Three repeated measurements were applied for each sample. The injectability assessment of the samples was applied on 24G (withdrawal) and 30G (injection) of needles. The results of viscosity and injectability of the samples are listed in Table 2 to Table 4.

As shown in Table 4, as the concentration of HA increased, the viscosity of HA/liposome-DSP composition also increased. The viscosity of the HA/liposome-DSP composition had an impact on its injectability. Therefore, an injectable hydrogel/liposome-drug composition has an indicated concentration range of hydrogel suitable for the present disclosure.

### Table 2 The viscosity of HA solutions

| HA concentration (w/v %) | Buffer for HA dissolution | Viscosity at 20 °C (mPa·S) |
|---|---|---|
| 0.228 | | 17.52 |
| 0.57 | HEPES/NaCl | 85.32 |
| 1.14 | | 2785.33 |

### Table 3 The viscosity of liposome-DSP compositions

| Sample | Phospholipid concentration (mM) | Viscosity at 20 °C (mPa·S) |
|---|---|---|
| L1 | 100 | 21 |
| L2 | 167 | 35 |

### Table 4 The viscosity and injectability of HA/liposome-DSP compositions

| Sample | HA concentration (w/v %) | Phospholipid concentration (mM) | Injectability | Viscosity at 20 °C (mPa·S) | Viscosity at 37 °C (mPa·S) |
|---|---|---|---|---|---|
| H1 | 0.0912 | | O | 19.35 | 8.6 |
| H2 | 0.228 | 60 | O | 44.28 | 29.5 |
| H3 | 0.456 | | O | 205.4 | 77.85 |
| H4 | 0.912 | | X | NA | NA |
| H5 | 0.228 | 100 | O | 98.12 | 58.5 |
| H6 | 0.456 | | O | NA | NA |

**Example 5 Dispersion behavior of the pharmaceutical compositions**

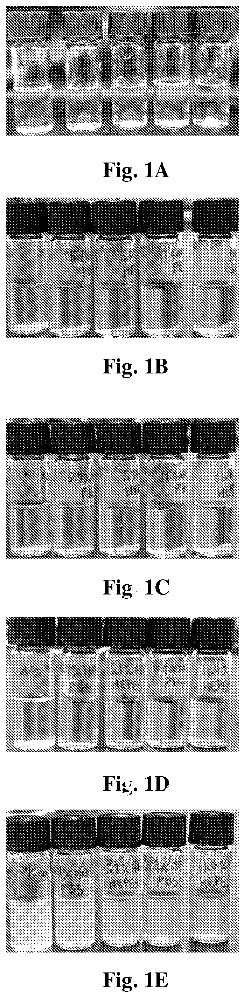

The dispersion behavior of the liposome-DSP composition and HA/liposome-DSP compositions in a medium was visually inspected. 30 μL of the liposome-DSP composition or 50 μL of the HA/liposome-DSP compositions was slowly added to 2 mL of saline in a transparent glass vial, where the total amounts of phospholipids in the liposome-DSP composition and the HA/liposome-DSP compositions were the same. The vials were incubated at 37°C and the dispersion behaviors of the samples were recorded at each indicated time point after incubation for (A) 0 h, (B) 1 h, (C) 2 h, (D) 3 h and (E) 24 h. Sample labels (from left to right) (1) Liposome-DSP composition; (2) 0.228% HA (PBS)/liposome-DSP composition; (3) 0.228%HA (HEPES/NaCl) /liposome-DSP composition; (4) 0.456% HA (PBS) /liposome-DSP composition; (5) 0.456% HA (HEPES/NaCl) /liposome-DSP composition. The samples were incubated at 37 °C and their dispersion behaviors were recorded by photograph at 0 hour (h), 1 h, 2 h, 3 h and 24 h. (Figures 1A-1E).

As illustrated in Figure 1E, after incubation of the samples for 24 hours without agitation/shaking, the liposome-DSP composition was completely dispersed in saline (sample 1), while the HA/liposome-DSP compositions all remained as small un-dispersed aggregates at the bottom of the vials. Besides, it was also observed that the higher the HA concentration in the HA/liposome-DSP composition, the less the spontaneous dispersion of the composition in saline, which may attribute to the viscosity of the composition.

**Example 6 Turbidity analysis**

Turbidity is a measure of the degree to which a solution loses its transparency due to the presence of dispersed particles. Here we measured the absorbance of the samples at λ = 400 - 700 nm (within the visible light range). According to the definition of absorbance (Equation 2), the higher the absorbance of a sample, the less the transmittance of it, which indicates the greater turbidity of the solution (more incident light is scattered, Equation 1). For the sample preparation, 50 μL of the liposome-DSP compositions (sample L1) or 50 μL of the HA/liposome-DSP compositions (sample H5 and H6) was added to 2 mL of saline, where the total amounts of phospholipids in the liposome-DSP composition and the HA/liposome-DSP compositions were the same. The samples were vibrated until the liposome-DSP or HA/liposome-DSP compositions were dispersed in saline completely. The homogeneous sample solutions were transferred to a 96-well plate (200 μL/well) and the absorbance measured at λ = 400 to 700 nm in a microplate reader (Synergy H1, BioTek).

$$\textit{Equation 1} \qquad \text{Transmitance (T)} = \frac{I}{I_0}$$

$$\textit{Equation 2} \qquad \text{Absorbance (A)} = \log_{10}\left(\frac{I_0}{I}\right) = -\log T$$

I: Transmitted light (output)
$I_0$: Incident light (input)

Figure 2A illustrates that in the wavelength range of 400 nm to 700 nm, the liposome-DSP composition exhibits higher absorbance spectrum than the HA/liposome-DSP compositions. The result has demonstrated an unexpected effect that after the compositions were completely dispersed in saline, the HA/liposome-DSP compositions displayed a reduced turbidity compared to the liposome-DSP composition. In addition, the higher the HA content in the composition, the better the turbidity-reducing effect. It is worth noting that the turbidity-reducing effect of the HA/liposome-DSP compositions covered the entire visible light range (400 nm - 700 nm) tested. In the day-light condition, human eyes are most sensitive to the wavelength of 555 nm. At wavelength of 555 nm, the turbidity of the liposome-DSP composition with 0.228% HA was about 21% lower compared to the liposome-DSP composition. The liposome-DSP composition with 0.456% HA reduced turbidity by about 37% compared to the liposome-DSP composition alone.

**Example 7 *In vitro* release (IVR) of the active agent**

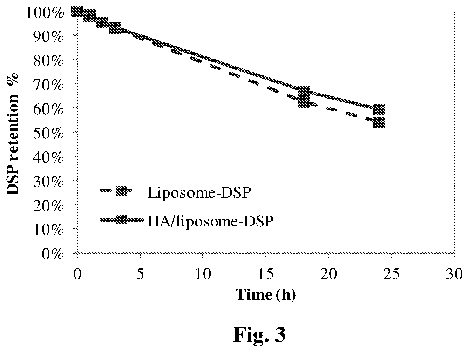

An *in vitro* evaluation of the active agent release from the liposome-drug composition and the hydrogel/liposome-drug composition was performed in a dissolution system containing a dialysis tube (Float-A-Lyzer®G2 Dialysis Device, 20 kDa, 1 mL, Spectra/Por®) as a sample reservoir surrounded by the release medium in a glass bottle. A sample reservoir was loaded with (1) 760 μL of saline and 240 μL of liposome-DSP composition (sample L1, where the composition was slowly added into the saline) or (2) 600 μL of saline and 400 μL of 0.456% HA/liposome-DSP composition (sample H3, where the composition was slowly added into the saline), where the total amounts of phospholipids and DSP in the sample reservoirs in the two groups were the same. Saline was used as the release medium in this *in vitro* release evaluation. The sample reservoir was placed in 200 mL of a release medium in a glass bottle, and the system was under a slow stir at 37 °C to facilitate the dissolution of DSP from the sample reservoir to the release medium. 1 mL of the release medium was collected at indicated time points, and the released amount of DSP was measured (UV, $\lambda$ = 241 nm) and calculated accordingly.
The IVR result in Figure 3 shows that the release profile of the HA/liposome-DSP composition was similar to the release profile of the liposome-DSP composition within 24 h. As liposome-DSP composition is known to be a sustained-release pharmaceutical composition, we concluded that the HA/liposome-DSP composition offered a comparable sustained release profile of the active agent from the pharmaceutical composition, compared to that of the liposome-DSP composition alone.

**Claims**

1.  A pharmaceutical composition for use in intravitreal administration, comprising:

    a lipid mixture comprising one or more lipids;
    an effective amount of an active agent or a pharmaceutically acceptable salt thereof; and
    a biocompatible hydrogel comprising hyaluronic acid,
    wherein the biocompatible hydrogel is present in the pharmaceutical composition at a concentration ranging from 0.01% (w/v) to 0.75% (w/v);
    wherein the active agent is an anti-angiogenesis agent or an anti-inflammatory agent, and

    wherein the pharmaceutical composition has a viscosity of 15 to 220 mPa·S at 20°C.

2.  The pharmaceutical composition of claim 1, wherein the biocompatible hydrogel has a molecular weight of about 4 kDa to about 8000 kDa.

3.  The pharmaceutical composition of any one of claims 1 to 2, wherein the biocompatible hydrogel is present in a concentration ranging from about 0.09% (w/v) to about 0.5% (w/v).

4. The pharmaceutical composition of any one of claims 1 to 3, wherein the lipid mixture comprises one or more phospholipids selected form the group consisting of dioleoylphosphatidylcholine (DOPC), and dioleoylphosphatidylglycerol (DOPG).

5. The pharmaceutical composition of claim 4, wherein the lipid mixture further comprises cholesterol.

6. The pharmaceutical composition of any one of claims 1 to 3, which is prepared by a process comprising:

    providing the lipid mixture and the active agent in a suspension; and
    mixing the suspension with a solution containing the biocompatible hydrogel, whereby a mixture of lipid nanoparticles and the biocompatible hydrogel forms.

7. The pharmaceutical composition of any one of claims 1 to 3, wherein the active agent is selected from the group consisting of bevacizumab, ranibizumab, aflibercept, brolucizumab, pegaptanib, cortisone, hydrocortisone, hydrocortisone acetate, tixocortol pivalate, fluocinolone, prednisolone, methylprednisolone, prednisone, triamcinolone acetonide, triamcinolone, mometasone, amcinonide, budesonide, desonide, fluocinonide, fluocinolone acetonide, halcinonide, betamethasone, betamethasone sodium phosphate, dexamethasone, dexamethasone sodium phosphate (DSP), fluocortolone, hydrocortisone-17-butyrate, hydrocortisone-17-valerate, alclometasone dipropionate, betamethasone valerate, betamethasone dipropionate, prednicarbate, clobetasone-17-butyrate, clobetasol-17-propionate, fluocortolone caproate, fluocortolone pivalate, fluprednidene acetate, difluprednate, loteprednol, fluorometholone, medrysone rimexolone, beclomethasone, cloprednol, cortivazol, deoxycortone, difluorocortolone, fluclorolone, fludrocortisone, flumethasone, flunisolide, flurandrenolone, meprednisone and paramethasone.

8. The pharmaceutical composition of any one of claims 1 to 3, wherein the anti-angiogenesis agent is an antagonist specific to VEGF selected from a group consisting of an antibody specific to VEGF, a VEGF receptor, a nucleic acid, and a small molecule.

9. The pharmaceutical composition of claim 8, wherein the antibody specific to VEGF is selected from a group consisting of a complete antibody molecule, Fab, Fab', $F(ab)_2$, $F(ab')_2$, scFv, di-scFv, scFv-Fc, single domain antibody, diabody, and triabody.

10. The pharmaceutical composition of any one of claims 1 to 3, wherein the anti-inflammatory agent is a corticosteroid.

11. The pharmaceutical composition of any one of claims 1 to 3, wherein the one or more lipids is at an amount ranging from about 2 $\mu$mol to about 200 $\mu$mol per mL of the pharmaceutical composition.

12. The pharmaceutical composition of any one of claims 1 to 3, wherein the one or more lipids is at an amount ranging from about 60 $\mu$mol to about 100 $\mu$mol per mL of the pharmaceutical composition.

13. The pharmaceutical composition of any one of claims 1 to 3, wherein the one or more lipids are at a concentration of 50 mM to 150 mM; the biocompatible hydrogel at an amount of 0.05% to 0.75% (w/v); and the pharmaceutical composition has a viscosity of 5 to 100 mPa·S at 37 °C.

14. A pharmaceutical composition of any one of claims 1 to 13 for use in reducing vitreous haze caused by intravitreal injection of a liposomal composition into vitreous humor of an eye of a subject in need thereof, wherein the liposomal composition comprises the active agent without the biocompatible hydrogel.

15. The pharmaceutical composition for use according to claim 14, wherein the subject in need thereof is suffering from at least one disorder selected from the group consisting of retinal diseases, macular degeneration, age-related macular degeneration, cystoid macular edema (CME), diabetic maculopathy, proliferative diabetic retinopathy (PDR), diabetic macular edema, retinal vein occlusions (RVO), neovascular glaucoma (NVG), retinal vascular tumors, choroidal hemangiomas, choroidal melanoma, vasoproliferative ocular tumor, Iris melanoma, radiation retinopathy, central serous chorioretinopathy (CSR), retinopathy of prematurity (ROP), endophthalmitis, uveitis, and retinitis.

**Patentansprüche**

1. Eine pharmazeutische Zusammensetzung zur intravitrealen Verabreichung, umfassend:

eine Lipidmischung, die ein oder mehrere Lipide umfasst;
eine effektive Menge eines Wirkstoffs oder eines pharmazeutisch akzeptablen Salzes davon und
ein biokompatibles Hydrogel, das Hyaluronsäure umfasst,
wobei das biokompatible Hydrogel in der pharmazeutischen Zusammensetzung in einer Konzentration von 0,01 % (Gewicht/Volumen) bis 0,75 % (Gewicht/Volumen) vorhanden ist;
wobei der Wirkstoff ein Anti-Angiogenese-Wirkstoff oder ein entzündungshemmender Wirkstoff ist, und
wobei die pharmazeutische Zusammensetzung eine Viskosität von 15 bis 220 mPa ·S bei 20 °C aufweist.

2. Die pharmazeutische Zusammensetzung nach Anspruch 1, wobei das biokompatible Hydrogel ein Molekulargewicht von etwa 4 kDa bis etwa 8000 kDa aufweist.

3. Die pharmazeutische Zusammensetzung nach einem der Ansprüche 1 bis 2, wobei das biokompatible Hydrogel in einer Konzentration von etwa 0,09 % (Gewicht/Volumen) bis etwa 0,5 % (Gewicht/Volumen) vorhanden ist.

4. Die pharmazeutische Zusammensetzung nach einem der Ansprüche 1 bis 3, wobei die Lipidmischung ein oder mehrere Phospholipide umfasst, die aus der Gruppe bestehend aus Dioleoylphosphatidylcholin (DOPC) und Dioleoylphosphatidylglycerol (DOPG) ausgewählt sind.

5. Die pharmazeutischeZusammensetzung nach Anspruch 4, wobei die Lipidmischung zudem Cholesterin umfasst.

6. Die pharmazeutischeZusammensetzung nach einem der Ansprüche 1 bis 3, die durch ein Verfahren hergestellt wird, das umfasst:

Bereitstellung der Lipidmischung und des Wirkstoffs in einer Suspension und
Mischen der Suspension mit einer Lösung, welche das biokompatible Hydrogel enthält, wodurch eine Mischung aus Lipid-Nanopartikeln und dem biokompatiblen Hydrogel entsteht.

7. Die pharmazeutische Zusammensetzung nach einem der Ansprüche 1 bis 3, wobei der Wirkstoff aus der Gruppe ausgewählt ist, welche besteht aus Bevacizumab, Ranibizumab, Aflibercept, Brolucizumab, Pegaptanib, Kortison, Hydrocortison, Hydrocortisonacetat, Tixocortolpivalat, Fluocinolon, Prednisolon, Methylprednisolon, Prednison, Triamcinolonacetonid, Triamcinolon, Mometason, Amcinonid, Budesonid, Desonid, Fluocinonid, Fluocinolonacetonid, Halcinonid, Betamethason, Betamethason-Natriumphosphat, Dexamethason, Dexamethason-Natriumphosphat (DSP), Fluocortolon, Hydrocortison-17-Butyrat, Hydrocortison-17-Valerat, Alclomethasondipropionat, Betamethasonvalerat, Betamethasondipropionat, Prednicarbat, Clobetason-17-Butyrat, Clobetasol-17-propionat, Fluocortolon-Caproat, Fluocortolon-Pivalat, Flupredniden-Acetat, Difluprednat, Loteprednol, Fluorometholon, MedrysonRimexolon, Beclomethason, Cloprednol, Cortivazol, Deoxycorton, Difluorocortolon, Fluclorolon, Fludrocortison, Flumethason, Flunisolid, Flurandrenolon, Meprednison und Paramethason.

8. Die pharmazeutische Zusammensetzung nach einem der Ansprüche 1 bis 3, wobei der Anti-Angiogenese-Wirkstoff ein spezifischer VEGF-Antagonist ist, der aus einer Gruppe ausgewählt wird, die einem VEGF-spezifischen Antikörper, einen VEGF-Rezeptor, eine Nukleinsäure und ein kleines Molekül umfasst.

9. Die pharmazeutische Zusammensetzung nach Anspruch 8, wobei der VEGF-spezifische Antikörper aus einer Gruppe ausgewählt wird, die ein vollständiges Antikörpermolekül, Fab, Fab', $F(ab)_2$, $F(ab')_2$, scFv, di-scFv, scFv-Fc, Single-Domain-Antikörper, Diabody und Triabody umfasst.

10. Die pharmazeutische Zusammensetzung nach einem der Ansprüche 1 bis 3, wobei der entzündungshemmende Wirkstoff ein Kortikosteroid ist.

11. Die pharmazeutische Zusammensetzung nach einem der Ansprüche 1 bis 3, wobei die ein oder mehrere Lipide in einer Menge von etwa 2 μmol bis etwa 200 μmol pro ml der pharmazeutischen Zusammensetzung enthalten sind.

12. Die pharmazeutische Zusammensetzung nach einem der Ansprüche 1 bis 3, wobei die ein oder mehrere Lipide in einer Menge von etwa 60 μmol bis etwa 100 μmol pro ml der pharmazeutischen Zusammensetzung enthalten sind.

13. Die pharmazeutische Zusammensetzung nach einem der Ansprüche 1 bis 3, wobei die ein oder mehrere Lipide in einer Konzentration von 50 mM bis 150 mM vorliegen, das biokompatible Hydrogel in einer Menge von 0,05 % bis 0,75 % (Gewicht/Volumen) und die pharmazeutische Zusammensetzung eine Viskosität von 5 bis 100 mPa·S bei 37 °C aufweist.

**14.** Die pharmazeutische Zusammensetzung nach einem der Ansprüche 1 bis 13 zur Verwendung für eine Verringerung von Glaskörpertrübungen, die durch intravitreale Injektion einer liposomalen Zusammensetzung in den Glaskörper eines Auges einer Person verursacht werden, welche diese benötigt, wobei die liposomale Zusammensetzung den Wirkstoff ohne das biokompatible Hydrogel umfasst.

**15.** Die pharmazeutische Zusammensetzung zur Verwendung gemäß Anspruch 14, wobei die Person, welche sie benötigt, an mindestens einer Erkrankung aus der Gruppe leidet, die Netzhauterkrankungen, Makuladegeneration, altersbedingte Makuladegeneration, zystoides Makulaödem (CME), diabetische Makulopathie, proliferative diabetische Retinopathie (PDR), diabetisches Makulaödem, retinale Venenverschlüsse (RVO), neovaskuläres Glaukom (NVG), retinale Gefäßtumoren, choroidale Hämangiome, Choroidalmelanom, vasoproliferativer Augentumor, Irismelanom, Strahlenretinopathie, zentrale seröse Chorioretinopathie (CSR), Frühgeborenen-Retinopathie (ROP), Endophthalmitis, Uveitis und Retinitis umfasst.

## Revendications

**1.** Composition pharmaceutique pour son utilisation pour une administration par voie intravitréenne, comprenant :

un mélange lipidique comprenant un ou plusieurs lipides ;
une quantité efficace d'un agent actif ou d'un sel pharmaceutiquement acceptable de celui-ci ; et
un hydrogel biocompatible comprenant de l'acide hyaluronique,
dans laquelle l'hydrogel biocompatible est présent dans la composition pharmaceutique à une concentration comprise entre 0,01 % (p/v) et 0,75 % (p/v) ;
dans laquelle l'agent actif est un agent anti-angiogénique ou un agent anti-inflammatoire , et

dans laquelle la composition pharmaceutique a une viscosité de 15 à 220 mPa·S à 20 °C.

**2.** Composition pharmaceutique selon la revendication 1, dans laquelle l'hydrogel biocompatible a un poids moléculaire d'environ 4 kDa à environ 8000 kDa.

**3.** Composition pharmaceutique selon l'une quelconque des revendications 1 à 2, dans laquelle l'hydrogel biocompatible est présent à une concentration comprise entre environ 0,09 % (p/v) et environ 0,5 % (p/v).

**4.** Composition pharmaceutique selon l'une quelconque des revendications 1 à 3, dans laquelle le mélange lipidique comprend un ou plusieurs phospholipides choisis dans le groupe constitué par la dioléoylphosphatidylcholine (DOPC) et le dioléoylphosphatidylglycérol (DOPG).

**5.** Composition pharmaceutique selon la revendication 4, dans laquelle le mélange lipidique comprend en outre du cholestérol.

**6.** Composition pharmaceutique selon l'une quelconque des revendications 1 à 3, qui est préparée par un procédé comprenant :

la fourniture du mélange lipidique et de l'agent actif dans une suspension ; et
le mélange de la suspension avec une solution contenant l'hydrogel biocompatible, permettant de former un mélange de nanoparticules lipidiques et d'hydrogel biocompatible.

**7.** Composition pharmaceutique selon l'une quelconque des revendications 1 à 3, dans laquelle l'agent actif est choisi dans le groupe constitué de bevacizumab, ranibizumab, aflibercept, brolucizumab, pegaptanib, cortisone, hydrocortisone, acétate d'hydrocortisone, pivalate de tixocortol, fluocinolone, prednisolone, méthylprednisolone, prednisone, acétonide de triamcinolone, triamcinolone, mométasone, amcinonide, budésonide, désonide, fluocinonide, acétonide de fluocinolone, halcinonide, bétaméthasone, phosphate sodique de bétaméthasone, dexaméthasone, phosphate sodique de dexaméthasone (DSP), fluocortolone, butyrate d'hydrocortisone-17, valérate d'hydrocortisone-17, dipropionate d'alclométasone, valérate de bétaméthasone, dipropionate de bétaméthasone, prednicarbate, butyrate de clobétasone-17, propionate de clobétasol-17, caproate de fluocortolone, pivalate de fluocortolone, acétate de fluprednidène, difluprednate, lotéprednol, fluorométholone, médrysone rimexolone, béclométhasone, cloprednol, cortivazol, désoxycortone, difluorocortolone, fluclorolone, fludrocortisone, fluméthasone, flunisolide, flurandrenolone, méprednisone et paraméthasone.

8. Composition pharmaceutique selon l'une quelconque des revendications 1 à 3, dans laquelle l'agent anti-angiogénique est un antagoniste spécifique du VEGF choisi dans le groupe constitué par un anticorps spécifique du VEGF, un récepteur du VEGF, un acide nucléique et une petite molécule.

9. Composition pharmaceutique selon la revendication 8, dans laquelle l'anticorps spécifique du VEGF est choisi dans le groupe constitué par une molécule d'anticorps complète, Fab, Fab', F(ab)$_2$, F(ab')$_2$, scFv, di-scFv, scFv-Fc, un anticorps à domaine unique, un diabody et un triabody.

10. Composition pharmaceutique selon l'une quelconque des revendications 1 à 3, dans laquelle l'agent anti-inflammatoire est un corticostéroïde.

11. Composition pharmaceutique selon l'une quelconque des revendications 1 à 3, dans laquelle le ou les lipides sont présents en une quantité comprise entre environ 2 $\mu$mol et environ 200 $\mu$mol par ml de composition pharmaceutique.

12. Composition pharmaceutique selon l'une quelconque des revendications 1 à 3, dans laquelle le ou les lipides sont présents en une quantité comprise entre environ 60 $\mu$mol et environ 100 $\mu$mol par ml de composition pharmaceutique.

13. Composition pharmaceutique selon l'une quelconque des revendications 1 à 3, dans laquelle le ou les lipides sont présents à une concentration comprise entre 50 mM et 150 mM ; l'hydrogel biocompatible est présent en une quantité comprise entre 0,05 % et 0,75 % (p/v) ; et la composition pharmaceutique a une viscosité comprise entre 5 et 100 mPa·S à 37 °C.

14. Composition pharmaceutique selon l'une quelconque des revendications 1 à 13, pour son utilisation pour réduire le voile vitré en causé par l'injection intravitréenne d'une composition liposomale dans l'humeur vitrée de l'œil d'un sujet qui en a besoin, dans laquelle la composition liposomale comprend l'agent actif sans l'hydrogel biocompatible.

15. Composition pharmaceutique pour son utilisation selon la revendication 14, dans laquelle le sujet qui en a besoin souffre d'au moins un trouble choisi dans le groupe constitué des maladies rétiniennes, dégénérescence maculaire, dégénérescence maculaire liée à l'âge, œdème maculaire cystoïde (OMC), maculopathie diabétique, rétinopathie diabétique proliférative (RDP), œdème maculaire diabétique, occlusions veineuses rétiniennes (OVR), glaucome néovasculaire (GNV), tumeurs vasculaires rétiniennes, hémangiomes choroïdiens, mélanome choroïdien, tumeur oculaire vasoproliférative, mélanome de l'iris, rétinopathie radique, choriorétinopathie séreuse centrale (CRSC), rétinopathie du prématuré (RDP), endophtalmie, uvéite et rétinite.

Fig. 1A

Fig. 1B

Fig. 1C

Fig. 1D

Fig. 1E

**Fig. 2A**

**Fig. 2B**

Fig. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4804539 A
- US 8956600 B
- US 2019151236 A1
- US 2014105960 A1

**Non-patent literature cited in the description**

- **M. ABRISHAMI et al.** *Retina*, May 2009, vol. 29 (5), 699-703
- **S K AKULA et al.** *Br J Ophthalmol.*, September 1994, vol. 78 (9), 677-680
- **G. ROUSER et al.** *Lipids*, 1970, vol. 5, 494-496
- **DEAMER et al.** *Acad. Sci.*, 1978, vol. 308, 250
- **BRUNNER et al.** *Biochim. Biophys. Acta*, 1976, vol. 455, 322
- **PICK et al.** *Arch. Biochim. Biophys.*, 1981, vol. 212, 186
- **SZOKA et al.** *Biochim. Biophys. Acta*, 1980, vol. 601, 559-71
- **HUANG et al.** *Biochemistry*, 1969, vol. 8, 344
- **KREMER et al.** *Biochemistry*, 1977, vol. 16, 3932
- **HOPE et al.** *Biochim. Biophys. Acta*, 1985, vol. 812, 55-65
- **BARENHOLZ et al.** *FEBS Lett.*, 1979, vol. 99, 210
- **SZOKA, F., JR. et al.** *Ann. Rev. Biophys. Bioeng.*, 1980, vol. 9, 467